# EUROPEAN PATENT SPECIFICATION

(11) **EP 1 367 387 B1**
(45) Date of publication and mention of the grant of the patent: **12.11.2014**
(21) Application number: 03011890.5
(22) Date of filing: 27.05.2003
(51) Int. Cl.: G01N 27/407, G01N 27/403

(54) **Solid electrolyte sensor for detecting a sulfurous component in a gas stream**
Festelektrolytsensor zur Detektion eines schwefelhaltigen Bestandteils in einem Gasstrom
Capteur à électrolyte solide pour la détection d'un composant sulfureux dans un flux de gaz

(30) Priority: 27.05.2002 JP 2002152939
(43) Date of publication of application: 03.12.2003
(73) Proprietor: SHINKO ELECTRIC INDUSTRIES CO. LTD., Nagano-shi, Nagano 380-0921 (JP)
(72) Inventor: Suganuma, Shigeaki, Oaza Kurita, Nagano-shi, Nagano 380-0921 (JP); Watanabe, Misa, Oaza Kurita, Nagano-shi, Nagano 380-0921 (JP); Horiuchi, Michio, Oaza Kurita, Nagano-shi, Nagano 380-0921 (JP)
(74) Representative: Patentanwälte Ruff, Wilhelm, Beier, Dauster & Partner

(56) References cited:
- DD-A1- 206 232
- JP-A- 8 068 775
- JP-A- 2000 325 790
- US-A- 5 429 727
- YATES C ET AL: "Anode materials for a hydrogen sulfide solid oxide fuel cell" JOURNAL OF THE ELECTROCHEMICAL SOCIETY, vol. 146, no. 8, August 1999 (1999-08), pages 2841-2844, XP002354620 ISSN: 0013-4651
- MIURA N ET AL: "Sensing characteristics and mechanism of hydrogen sulfide sensor using stabilized zirconia and oxide sensing electrode" SENSORS AND ACTUATORS B, vol. 34, no. 1-3, August 1996 (1996-08), pages 367-372, XP004078007 ISSN: 0925-4005
- SUTHERLAND F M ET AL: "High temperature measurements on solid and gaseous sulphide systems" SOLID STATE IONICS, vol. 53-56, no. PART 1, 1 July 1992 (1992-07-01), pages 68-74, XP000399104 ISSN: 0167-2738

## Description

### BACKGROUND OF THE INVENTION

### 1. Field of the Invention

The invention relates to a sensor and a device for detecting sulfur and, more particularly, to a sensor and a device for detecting a sulfurous component contained in a substantially oxygen-free gas stream.

### 2. Description of the Related Art

Sensors and method for detecting hydrogen sulfide are for example described in JP 08-68775, DD 206 232, as well as in the Journal articles "Anode Materials for a Hydrogen Sulfide Solid Oxide Fuel Cell" by Chris Yates and Jack Winnick (Journal of the Electrochemical Society, p. 2841-2844, and "Sensing characteristics and mechanism of hydrogen sulphide sensor using stabilized zirconia and oxide sensing electrode", N. Miura et al./Sensors and Actuators B34 (1996) p. 367-372). JP 2000/325790 A discloses the use of a rhodium oxide (Rh203) wash coat for the catalytic oxidation of H2S.

Sensors for measuring a concentration of sulfurous component in a sulfur-containing gas stream, such as a flue gas exhausted from a flue of a boiler, are further proposed in, for example, JP 9-80017 A and JP 9-80018.

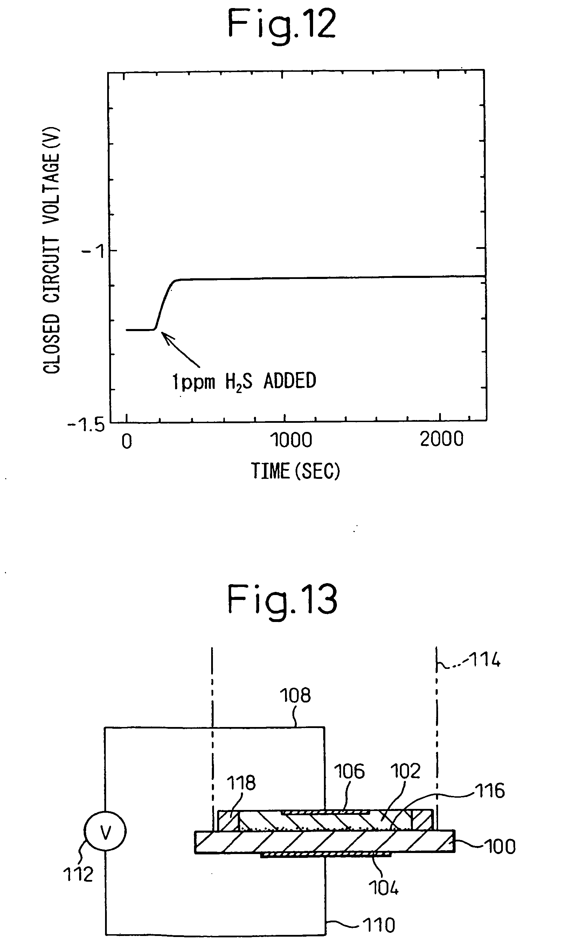

A sensor proposed in the above documents is shown in Fig. 13. In the sensor shown in Fig. 13, a detecting electrode 102 made of a sulfate, including silver sulfate, is formed within a frame 118 provided on one side of a solid electrolyte substrate 100 (sometimes simply called substrate 100 hereinafter) made of yttria-stabilized zirconia (YSZ) solid electrolyte material. In addition, a platinum electrode 104, as a reference electrode, is formed on the other side of the substrate 100, and a silver-containing electrode 106 is formed on the surface of the detecting electrode 102. The platinum electrode 104 and the silver-containing electrode 106 are connected to a voltmeter 112 via platinum wires 110 and 108. At the interface between the detecting electrode 102 and the substrate 100, platinum 116, which is produced by the pyrolysis of a platinum compound, is present.

The portion including the detecting electrode 102 of the sensor shown in Fig. 13 is inserted in a pipe 114, into which a flue gas stream is introduced, and the portion including the platinum electrode 104 is located outside the pipe 114.

When a flue gas stream containing sulfur dioxide gas is introduced into the pipe 114 of the sensor shown in Fig. 13, an electromotive force is generated resulting from electrochemical reactions occurring in the system of the platinum electrode 104, the substrate 100, the detecting electrode 102 and the silver-containing electrode 106. By measuring the electromotive force, the sulfur in the flue gas stream can be detected.

Although the sensor shown in Fig. 13 can detect sulfur in an oxygen-containing gas stream, such as an exhaust gas stream from a flue of a boiler, it cannot detect sulfur in a substantially oxygen-free gas stream, such as sulfur in a gas stream processed in a reforming process for reforming a commercial fuel gas or gasoline to feed hydrogen gas to a fuel cell for domestic or automobile use.

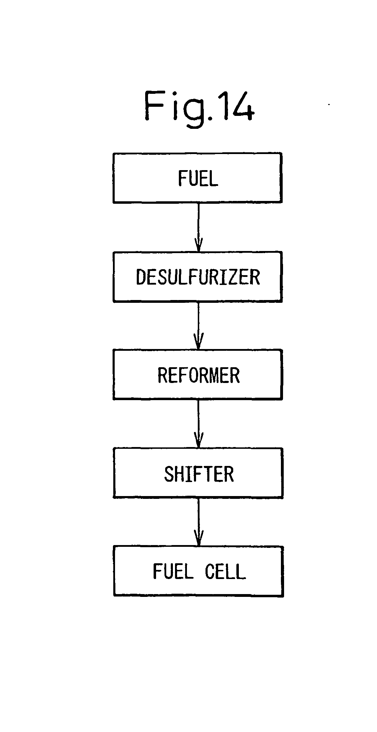

Such a reforming process is illustrated in Fig. 14. In this reforming process, a gasified fuel stream is fed to a desulfurizer to remove sulfur compounds therefrom, in order to remove the sulfur compounds contained in a gasoline which contains sulfur compounds originated from a crude oil, or a commercial fuel gas to which a sulfur compound is added to give it an unpleasant smell. The fuel gas stream, from which the sulfur compounds are removed, is then hydrogenated in a reformer, and the hydrogen is passed through a shifter, in which contained CO is converted to CO₂, and is fed to a fuel cell.

When the absorbing capability of a desulfurization catalyst or the like filled in the desulfurizer is reduced, a poorly desulfurized fuel gas stream is fed to the reformer and the shifter, to thereby reduce the functions of the reformer and the shifter, and to further reduce the power generation capacity of the fuel cell. For this reason, it is required that a measuring means, which can detect the sulfur in the fuel gas stream desulfurized in the desulfurizer, is provided between the desulfurizer and the reformer. Such a measuring means must be a measuring means capable of continuously detect sulfurous components in a substantially oxygen-free gas stream.

Even if the fuel gas stream desulfurized in the desulfurizer has a small content of sulfur compounds capable of retaining the functions of the reformer and the sifter, the functions of the reformer and the shifter are gradually reduced depending on accumulated amounts of sulfur compounds when the fuel gas stream containing the sulfur compounds is continually fed. For this reason, it is also necessary to measure the accumulated amounts of sulfur compounds fed to the reformer and the shifter.

### SUMMARY OF THE INVENTION

It is an object of the invention to provide a sulfur-detecting sensor and a sulfur-detecting device which can detect a sulfur compound or compounds in a substantially oxygen-free gas stream.

To this end, the inventors have investigated hydrogen gas streams containing hydrogen sulfide (H₂S).

Through the investigation, they have discovered that, by providing on one side of a solid electrolyte substrate made of a fired body of yttria-stabilized zirconia with a detecting electrode to be contacted with a hydrogen gas stream, providing on the other side of the solid electrolyte substrate with a reference electrode to be contacted with air, and forming the detecting electrode from a metal having a high reactivity with hydrogen sulfide, a difference in potential induced between the detecting electrode and the reference electrode by the electrochemical reaction of oxygen ion moved through the solid electrolyte substrate with hydrogen in the hydrogen gas stream is changed by the reaction of the hydrogen sulfide with the metal forming the detecting electrode when the hydrogen gas stream includes hydrogen sulfide, and have produced the invention.

More specifically, the invention resides in a sulfur-detecting sensor for detecting a sulfurous component contained in a substantially oxygen-free gas stream, the sensor being characterized by comprising a solid electrolyte substrate through which oxygen ions can move, and electrodes for detecting a difference in potential induced by a electrochemical reaction of oxygen ions moving through the solid electrolyte substrate with a component constituting the gas stream, which electrodes for detecting a difference in potential include a detecting electrode formed on one side of the solid electrolyte substrate and brought into contact with the gas stream, and a reference electrode formed on the other side of the solid electrolyte substrate and brought into contact with an oxygen-containing fluid, the detecting electrode being formed from a metal selected from the group consisting of Ni, Cu, and Zn, or an oxide thereof.

In the invention, by forming the detecting electrode from a transition metal having an enthalpy of formation of oxide of not smaller than -650 kJ/mol at 25°C, or an oxide thereof, the change in potential difference between the detecting electrode and the reference electrode caused by a reaction of the sulfurous component contained in the gas stream with the metal forming the detecting electrode can be definitely known in the case where the potential difference between the detecting electrode and the reference electrode is measured by a closed circuit, as described below.

It is preferred for the detecting electrode formed from such a metal to contain platinum, which can accelerate the reaction rate in the detecting electrode.

On the other hand, forming the reference electrode from a metal material containing platinum makes it easy to convert oxygen in the air to oxygen ions.

As the solid electrolyte substrate, a sintered body of yttria-stabilized zirconia, a sintered body of scandia-stabilized zirconia, a sintered body of ceria to which samaria is added, or a sintered body of scandia-stabilized zirconia to which ceria is added, can be suitably used.

The invention also resides in a sulfur-detecting device for detecting a sulfurous component contained in a substantially oxygen-free gas stream, characterized by comprising the aforementioned sulfur-detecting sensor, and further comprising a voltmeter for measuring a potential difference between the detecting electrode and the reference electrode of the sulfur-detecting sensor.

In the invention, by using, as a circuit made of the solid electrolyte substrate, the detecting electrode, the reference electrode and the voltmeter, a closed circuit to which an electric current is supplied from a unit generating a constant current so that the current density at the detecting electrode is at a constant value, the amount of current between the detecting electrode and the reference electrode can be constant, and the change in potential difference between the detecting electrode and the reference electrode caused by a reaction of the sulfurous component contained in the gas stream with the metal forming the detecting electrode can be definitely known.

Also, by using, as a circuit made of the solid electrolyte substrate, the detecting electrode, the reference electrode and the voltmeter, an open circuit to which no current is externally supplied, an inexpensive sulfur-detecting device can be provided.

According to the sulfur-detecting sensor of the invention, a sulfurous component contained in a substantially oxygen-free gas stream can be detected. Although the basis for this has not yet been precisely known, it is considered to be as follows.

The sulfur-detecting device of the invention is provided with a solid electrolyte substrate through which oxygen ions are capable of moving, a detecting electrode formed on one side of the solid electrolyte substrate and brought into contact with a gas stream, and a reference electrode formed on the other side of the solid electrolyte substrate and brought into contact with an oxygen-containing fluid. On this account, an electromotive force is generated in the detecting electrode, by an electrochemical reaction between oxygen ion moved through the solid electrolyte substrate and a component of the gas stream, resulting in the occurence of potential difference between the detecting electrode and the reference electrode. The detecting electrode is formed from a metal which easily reacts with a sulfurous component. Accordingly, when a sulfurous component is contained in the gas stream, a sulfide or the like is readily formed in the detecting electrode, and inhibits the electrochemical reaction in the detecting electrode, resulting in the change in potential difference occurring between the detecting electrode and the reference electrode. Consequently, by measuring the amount of change in potential difference, the amount of sulfurous component in the gas stream can be measured.

When a sulfide or the like produced by the reaction of a metal forming the detecting electrode with a sulfurous component in the gas stream cannot be scattered into the gas stream or to be reduced, the sulfide is accumulated in the detecting electrode. Therefore, by measuring the change in potential difference between the detecting electrode and the reference electrode after a lapse of predetermined time, an integrated amount of sulfurous component in the gas stream having passed over the sensor in the predetermined time can be determined.

### BRIEF DESCRIPTION OF THE DRAWINGS

The above and other objects and advantages of the invention will be well understood and appreciated by a person with ordinary skill in the art, from consideration of the following detailed description made by referring to the attached drawings, wherein:
Fig. 1 schematically illustrates first embodiments of the sulfur-detecting sensor and the sulfur-detecting device of the invention;
Fig. 2 schematically illustrates second embodiments of the sulfur-detecting sensor and the sulfur-detecting device of the invention;
Fig. 3 shows a change in potential difference between the detecting electrode and the reference electrode of a sulfur-detecting sensor obtained for a hydrogen sulfide-containing gas using the sulfur-detecting device shown in Fig. 1;
Fig. 4 shows another change in potential difference between the detecting electrode and the reference electrode of a sulfur-detecting sensor obtained for a hydrogen sulfide-containing gas using the sulfur-detecting device shown in Fig. 1;
Fig. 5 shows a further change in potential difference between the detecting electrode and the reference electrode of a sulfur-detecting sensor obtained for a hydrogen sulfide-containing gas using the sulfur-detecting device shown in Fig. 1;
Fig. 6 shows a still further change in potential difference between the detecting electrode and the reference electrode of a sulfur-detecting sensor obtained for a hydrogen sulfide-containing gas using the sulfur-detecting device shown in Fig. 1;
Fig. 7 shows another change in potential difference between the detecting electrode and the reference electrode of a sulfur-detecting sensor obtained for a hydrogen sulfide-containing gas using the sulfur-detecting device shown in Fig. 1;
Fig. 8 shows a further change in potential difference between the detecting electrode and the reference electrode of a sulfur-detecting sensor obtained for a hydrogen sulfide-containing gas using the sulfur-detecting device shown in Fig. 1;
Fig. 9 is a graph showing changes in potential differences between the detecting electrodes and the reference electrodes of sulfur-detecting sensors obtained for a concentration of hydrogen sulfide in a hydrogen sulfide-containing gas using the sulfur-detecting device shown in Fig. 1;
Fig. 10 shows a change in potential difference between the detecting electrode and the reference electrode of a sulfur-detecting sensor obtained for a hydrogen sulfide-containing gas using the sulfur-detecting device shown in Fig. 2;
Fig. 11 shows another change in potential difference between the detecting electrode and the reference electrode of a sulfur-detecting sensor obtained for a hydrogen sulfide-containing gas using the sulfur-detecting device shown in Fig. 2;
Fig. 12 shows a further change in potential difference between the detecting electrode and the reference electrode of a sulfur-detecting sensor obtained for a hydrogen sulfide-containing gas using the sulfur-detecting device shown in Fig. 2;
Fig. 13 schematically illustrates a prior sulfur-detecting sensor and a prior sulfur-detecting device; and
Fig. 14 illustrates a reforming process for feeding a fuel gas stream to a fuel cell.

### DETAILED DESCRIPTION OF THE INVENTION

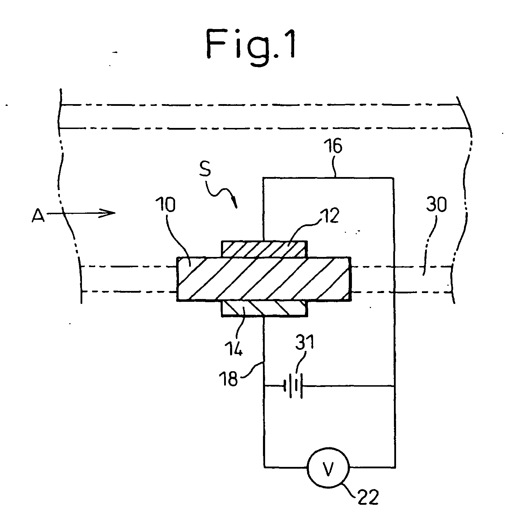

Fig. 1 shows an embodiment of the sulfur-detecting device of the invention. A sulfur-detecting sensor S constituting the sulfur-detecting device shown in Fig. 1 uses, as a solid electrolyte substrate 10 (sometimes simply called substrate 10 hereinafter), a sintered body of yttria-stabilized zirconia (YSZ) solid electrolyte material. A detecting electrode 12 is provided on one side of the substrate 10, and a reference electrode 14 made from platinum is provided on the other side of the substrate 10.

Each of the detecting electrode 12 and the reference electrode 14 of the sulfur-detecting sensor S is connected with one end of each of platinum wires 16 and 18, and the other end of each of the platinum wires 16 and 18 is connected to a voltmeter 22 for measuring a potential difference between the detecting electrode 12 and the reference electrode 14. The wires 16 and 18 are supplied, in the midst thereof, with a current from a unit 31 generating a constant current, so as to make the current density in the detecting electrode 12 constant. On this account, the circuit made up of the substrate 10, the detecting electrode 12, the reference electrode 14, and the voltmeter 22 forms a closed circuit.

The detecting electrode 12 in the sulfur-detecting sensor S shown in Fig. 1 is formed from a metal which easily reacts with a sulfurous component in a gas stream. Specifically, the detecting electrode 12 is formed from a transition metal having an enthalpy of formation of sulfide at 25°C of not smaller than -250 kJ/mol (preferably not smaller than -210 kJ/mol, and particularly preferably not smaller than -206 kJ/mol), or an oxide thereof. If the detecting electrode 12 is made from a transition metal having an enthalpy of formation of sulfide at 25°C of smaller than -250 kJ/mol, or an oxide thereof, the sulfur-detecting sensor S has a sensitivity, for a concentration of sulfurous component contained in a gas stream, which tends to be low.

Although it is not necessary to specify the upper limit of the enthalpy of formation of sulfide at 25°C, it may be less than 0 kJ/mol. In other words, the transition metal for the detecting electrode may have an enthalpy of formation of sulfide at 25°C of not smaller than -250 kJ/mol and less than 0 kJ/mol, in general.

Furthermore, it is preferred for the metal forming the detecting electrode 12 to be a transition metal having an enthalpy of formation of oxide at 25°C of not smaller than -650 kJ/mol (preferably not smaller than -600 kJ/mol, and especially not smaller than -581 kJ/mol), or an oxide thereof. Although it is also not necessary to specify the upper limit of the enthalpy of formation of oxide at 25°C, it may be less than 0 kJ/mol. In other words, the transition metal may have an enthalpy of formation of oxide at 25°C of not smaller than -650 kJ/mol and less than 0 kJ/mol, in general.

The metal for the formation of the detecting electrode 12 is a metal selected from the group consisting of , nickel (Ni), copper (Cu), and zinc (Zn), or an oxide thereof.

The detecting electrode 12 formed from such a metal material may contain another metal which is chemically stable at an operating temperature and in an operating atmosphere of the sulfur-detecting sensor S shown in Fig. 1. Particularly, platinum can accelerate a reaction rate in the detecting electrode 12, which is preferred.

For the fabrication of the sulfur-detecting sensor S shown in Fig. 1, a substrate 10 made of a solid electrolyte material, such as yttia-stabilized zirconia (YSZ), is first formed by firing at a given temperature, after which a detecting electrode 12 is formed by applying a paste, in which a material for the formation of the detecting electrode 12, such as silver, is blended, onto one side of the substrate 10, and firing it. Subsequently, a reference electrode 14 is formed on the other side of the substrate 10 by applying a platinum paste thereonto prior to firing..

Each of the detecting electrode 12 and the reference electrode 14 of the sulfur-detecting sensor S thus formed is connected to one end of each of wires 16 and 18 of platinum, and a voltmeter 22 is connected to the respective other ends of the wires 16 and 18. The wires 16 and 18 are connected, in the midst thereof, with a constant-current generating unit 31, which supplies an electrical current so as to make the current density in the detecting electrode 12 constant.

Subsequently, the sulfur-detecting sensor S is installed into a pipe 30 through which a gas stream containing a sulfurous component flows in the direction of the arrow A, as shown in Fig. 1, in such a manner that the detecting electrode 12 is brought into contact with the gas stream containing a sulfurous component, and the reference electrode 14 is brought into contact with the air.

When the solid electrolyte material forming the substrate 10 is heated to a temperature at which it can display ion conductivity while causing a substantially oxygen-free gas stream to flow through the pipe 30 in the direction of the Arrow A, oxygen in the air outside the pipe 30 is ionized in the presence of the platinum catalyst of the reference electrode 14 to form oxygen ions (O²⁻), which move through the substrate 10 to reach the detecting electrode 12.

In the detecting electrode 12, an electromotive force is generated by an electrochemical reaction of the component of the gas stream diffused through the detecting electrode 12 with the oxygen ion (O²⁻) moving through the substrate 10, resulting in the occurrence of a potential difference between the detecting electrode 12 and the reference electrode 14. The resultant potential difference can be measured by the voltmeter 22.

When the gas stream contains a sulfurous component, the sulfurous component diffused into the detecting electrode 12, formed from a metal which easily reacts with the sulfurous component, will react with the metal forming the detecting electrode 12 to produce a sulfide or the like. The sulfide or the like produced blocks the electrochemical reaction in the detecting electrode 12 of the component of the gas stream with the oxygen ion (O²⁻) moving through the substrate 10, to thereby increase the internal resistance of the sulfur-detecting sensor S, resulting in the change in potential difference generated between the detecting electrode 12 and the reference electrode 14. Accordingly, the concentration of sulfurous component in the gas stream can be determined by measuring the change in potential difference generated between the detecting electrode 12 and the reference electrode 14.

Although the mechanism of the change in potential difference between the detecting electrode 12 and the reference electrode 14 has not been elucidated in detail, it is inferred to be in the case where a stream of hydrogen gas is used as a substantially oxygen-free gas stream, and hydrogen sulfide (H₂S), as a sulfurous component, is mixed in the steam of hydrogen gas, as follows.

The hydrogen of the hydrogen sulfide (H₂S) diffused through the detecting electrode 12 reacts with oxygen ion (O²⁻) moving through the substrate 10 to yield sulfur, and the resultant sulfur is then adsorbed by the metal forming the detecting electrode 12, or reacts with the metal forming the detecting electrode 12 to form a sulfide. The sulfur adsorbed by the metal or the sulfide formed (sometimes called a sulfide or the like hereinafter) blocks the electrochemical reaction of hydrogen with oxygen ion (O²⁻) in the detecting electrode 12 to thereby lower the electromotive force, so that the potential difference between the detecting electrode 12 and the reference electrode 14 is lowered.

To definitely represent such an effect of a sulfurous component by the potential difference between the detecting electrode 12 and the reference electrode 14, it is necessary to stabilize the rate of electrochemical reaction in the detecting electrode 12. In the sulfur-detecting device shown in Fig. 1, the rate of electrochemical reaction in the detecting electrode 12 can be stabilized by supplying a current by the constant-current generating unit 31 provided in the midst of the loop of the wires 16, 18, in such a manner that the current density in the detecting electrode 12 is constant.

As a result, even if portions (or areas) contributing to the electrochemical reaction in the detecting electrode 12 are decreased by the formation of a sulfide or the like in the detecting electrode 12, the amount of current supplied is adjusted by the constant-current generating unit 31 in such a manner that the current density in the detecting electrode 12 is constant. On this account, the electrochemical reaction smoothly proceeds in the portions capable of contributing to the electrochemical reaction in the detecting electrode 12. Consequently, a potential difference can be stably created between the detecting electrode 12 and the reference electrode 14, depending on the portions (or areas) capable of contributing to the electrochemical reaction in the detecting electrode 12.

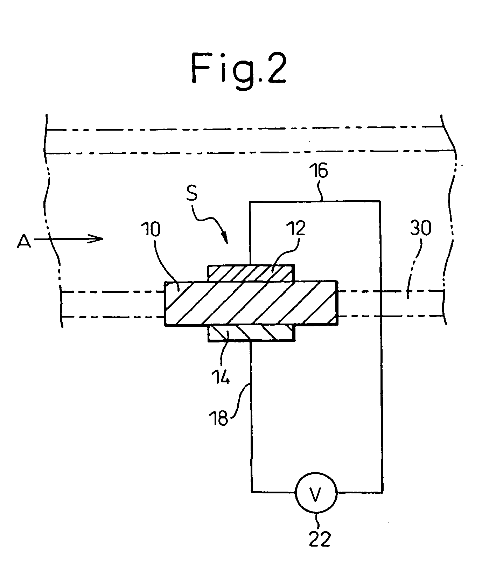

Thus, the rate of electrochemical reaction in the detecting electrode 12 is stabilized by the provision of constant-current generating unit 31 in the sulfur-detecting device shown in Fig. 1. On the other hand, in the case where the electrochemical reaction proceeds in the detecting electrode 12 without the provision of constant-current generating unit 31, a sulfur-detecting device shown in Fig. 2 can be used.

In the sulfur-detecting device shown in Fig. 2, a substrate 12, a detecting electrode 12, a reference electrode 14, and a voltmeter 22 form an open circuit to which a current is not externally supplied. On this account, the sulfur-detecting device shown in Fig. 2 has a simple structure compared to the sulfur-detecting device shown in Fig. 1 having the constant-current generating unit 31 provided.

The members in Fig. 2 which are the same as those of the sulfur-detecting device shown in Fig. 1 are identified by the same reference code as in Fig. 1, and detailed descriptions thereof are skipped.

Of the metals used for the detecting electrode 12 of the sulfur-detecting sensor S for the sulfur-detecting devices shown in Figs. 1 and 2, metals, the formation of sulfide of which, or the adsorption of a sulfurous component to which, reaches equilibrium in a short time while the formed sulfide of which or the sulfurous component adsorbed to which is lost with a lapse of time, are suitable for applications in which momentary change in concentration of a sulfurous component in a gas stream is measured. Specifically, such metals include rhodium (Rh) and nickel (Ni), and oxides thereof can be used.

Also, metals, the formation of sulfide of which, or the adsorption of a sulfurous component to which, takes a long time until it reaches equilibrium while the formed sulfide of which or the sulfurous component adsorbed to which is hard to lose, are suitable for applications in which an accumulated amount of sulfurous component in a gas stream having passed over a detecting electrode made from the metal is measured. Such metals include copper (Cu) and zinc(Zn), and oxides thereof may be used.

In the sulfur-detecting sensor S used in the sulfur-detecting device depicted in Figs. 1 and 2, the detecting electrode 12 is formed from a metal which easily reacts with a sulfurous component. On this account, when a gas stream contains a sulfurous component, a sulfide or the like is easily formed in the detecting electrode 12, and inhibits an electrochemical reaction in the detecting electrode 12, resulting in the change in potential difference occurring between the detecting electrode 12 and the reference electrode 14. Consequently, by measuring an amount of the change in potential difference, an amount of sulfurous component in a gas stream can be measured and, as a result, this makes it possible to measure an amount of sulfurous component in a gas stream in a substantially oxygen-free state, which could not measured by a conventional sulfur-detecting sensor.

In the sulfur-detecting sensors shown in Figs. 1 and 2, although the sintered body of yttria-stabilized zirconia is used as the substrate 10, it may be replaced with a sintered body of scandia-stabilized zirconia, a sintered body of ceria to which samaria is added, or a sintered body of scandia-stabilized zirconia to which ceria is added.

### EXAMPLES

The following examples further describe the invention in detail.

### Example 1 [Comparative example]

A sulfur-detecting sensor S shown in Fig. 1 was made by applying a mixed paste of rhodium oxide and a platinum paste in a volume ratio of 1:2 to 1:3 on one side of a substrate 10 made from yttria-stabilized zirconia (YSZ) solid electrolyte material, applying a platinum paste on the other side of the substrate 10, and then firing the applied materials. The resultant sulfur-detecting sensor S had a substrate 10, one side of which a porous detecting electrode 12 of mixed rhodium oxide and platinum was formed, and the other side of which a porous reference electrode 14 of platinum was formed.

A sulfur-detecting device as shown in Fig. 1 was then fabricated using the resultant sulfur-detecting sensor S. In the sulfur-detecting device shown in Fig. 1, the detecting electrode 12 and the reference electrode 14 are connected with a voltmeter 22 through wires 16 and 18, respectively, and each of wires 16 and 18 is connected with a constant-current generating unit 31 in the midst thereof. The constant-current generating unit 31 supplies a current to a circuit consisting of the Substrate 10, the detecting electrode 12, the reference electrode 14 and the voltmeter 22, in such a manner that the current density in the detecting electrode 12 is constant.

The sulfur-detecting sensor S was installed to a pipe 30 as shown in Fig. 1, and was placed in an electric furnace (not shown) along with the pipe 30, while the voltmeter 22 and the constant-current generating unit 31 were outside the furnace. A stream of hydrogen gas (hydrogen gas concentration of 4% by volume, and nitrogen gas concentration of 96% by volume) was introduced in the pipe 30 in the direction of the arrow A to be passed over the detecting electrode 12, and air was passed over the reference electrode 14 outside the pipe 30, while the atmosphere in the furnace was heated to 600°C at which the substrate 10 develops ion conductivity. During that time, the constant-current generating unit 31 supplied a current to the circuit of the substrate 10, the detecting electrode 12, the reference electrode 14 and the voltmeter 22, so that the current density in the detecting electrode 12 was 1600 microamperes per square centimeter.

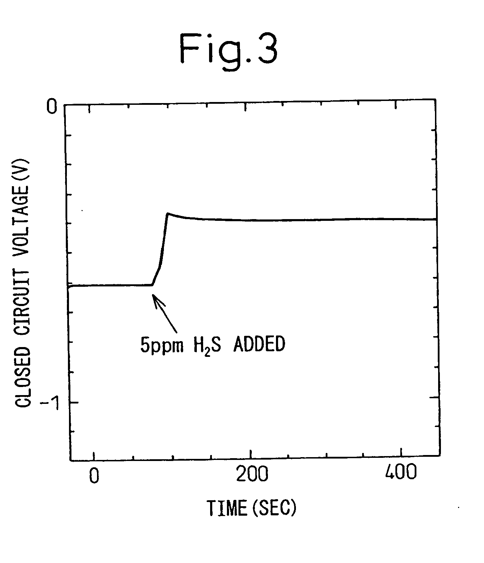

The potential difference between the detecting electrode 12 and the reference electrode 14 measured by the voltmeter 22 under the above conditions was about -0.6 volt, as shown as a closed circuit voltage in Fig. 3.

Subsequently, when H₂S was introduced at the side of the detecting electrode 12 in a concentration of 5 ppm relative to the stream of hydrogen gas, the closed circuit voltage measured by the voltmeter 22 was dramatically increased to about -0.38 volt (i.e., the potential difference was decreased), as shown in Fig. 3. It is inferred that this was caused by the occurrence of a sulfide or the like from rhodium oxide in the detecting electrode 12, which blocked the electrochemical reaction of hydrogen with oxygen ion (O²⁻), resulting in the decrease in electromotive force.

Thus, the sulfur-detecting sensor showing a characteristic represented in Fig. 3 is suitable for a sensor for measuring the change in H₂S concentration in a hydrogen gas stream.

### Example 2

Sulfur-detecting sensors were made in the same manner as in Example 1 except that the rhodium oxide in Example 1 was replaced with nickel oxide, copper, zinc oxide, tin oxide and silver, respectively. Table 1 summarizes enthalpies of formation of sulfide at 25°C and enthalpies of formation of oxide at 25°C of these metals.

Closed circuit voltages of the resultant sulfur-detecting sensors were determined as in Example 1. Results are summarized in Table 1. For the determination of closed circuit voltage, the constant-current generating unit 31 (Fig. 1) supplied a current to the circuit of the substrate 10, the detecting 12, the reference electrode 14 and the voltmeter 22 so that the detecting electrode 12 had a current density shown in Table 1.

**Table 1**

| | Sulfide Formation | Oxide Formation | Current Density |
|---|---|---|---|
| Metals | Enthalpies (kJ/mol) | Enthalpies (kJ/mol) | (µA/cm²) |
| Nickel | -82 (NiS) | -240 (NiO) | 350 |
| Copper | -53 (CuS) | -169 (Cu₂O) | 350 |
| Silver | -33 (Ag₂S) | -31 (Ag₂O) | 350 |
| Zinc | -206 (ZnS) | -348 (ZnO) | 3.5 |
| Tin | -100 (SnS) | -581 (SnO₂) | 180 |

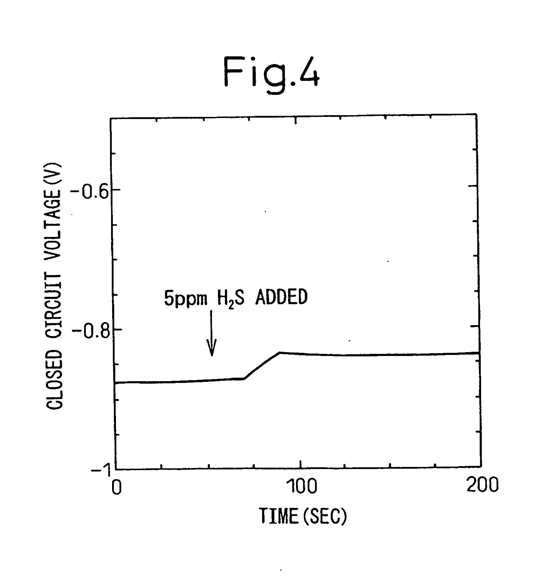

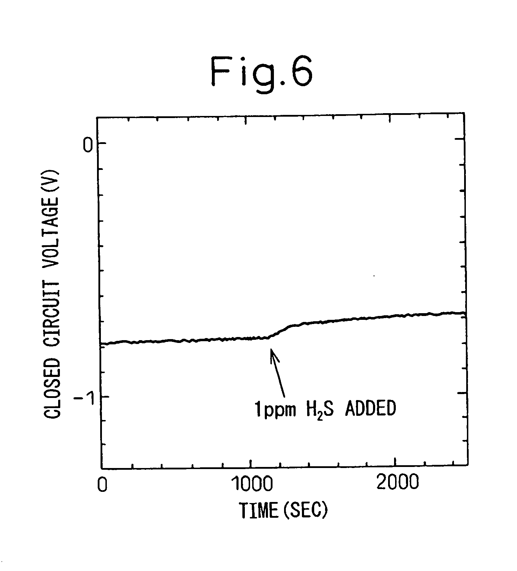

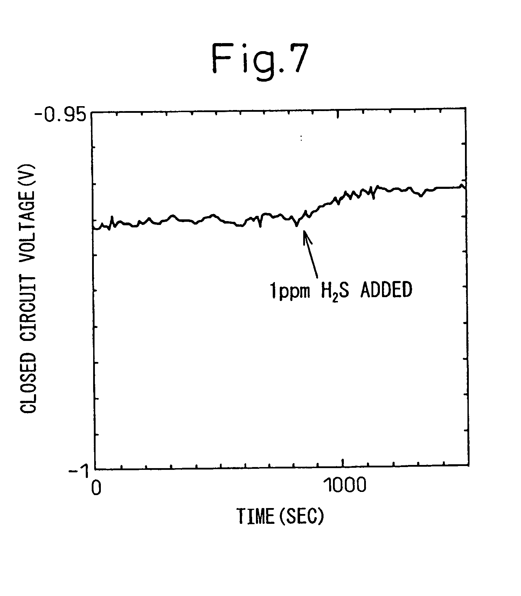

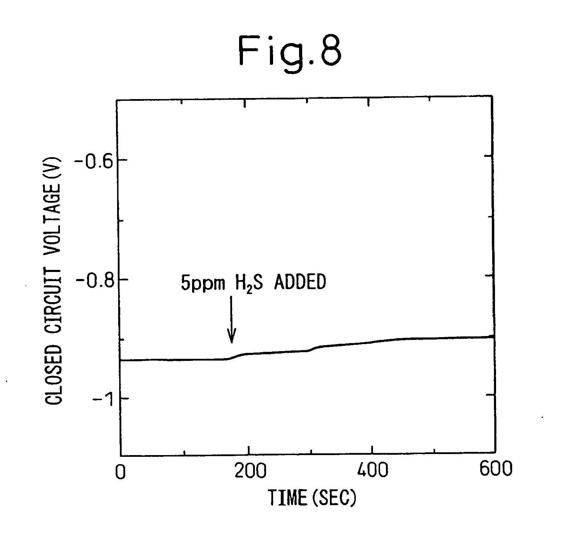

A concentration of H₂S contained in the stream of hydrogen (hydrogen gas concentration of 4% by volume, and nitrogen gas concentration of 96% by volume) was 5 ppm or 1 ppm. Figs. 4 to 8 specify the concentrations of H₂S used.

As seen in Figs. 4 to 8, all of the sulfur-detecting devices showed increased closed circuit voltages (decreased potential differences) with the introduction of H₂S to the pipe 30 (Fig. 1).

However, in the sulfur-detecting sensor using nickel oxide, the measured result of which is shown in Fig. 4, the closed circuit voltage was dramatically increased with the introduction of H₂S as in the sulfur-detecting sensor using rhodium oxide, the measured result of which is shown in Fig. 3.

Thus, the sulfur-detecting sensor showing the characteristic represented in Fig. 4 is suitable for a sensor for measuring the change in H2S concentration in a hydrogen gas stream, as is the sulfur-detecting sensor showing the characteristic represented in Fig. 3.

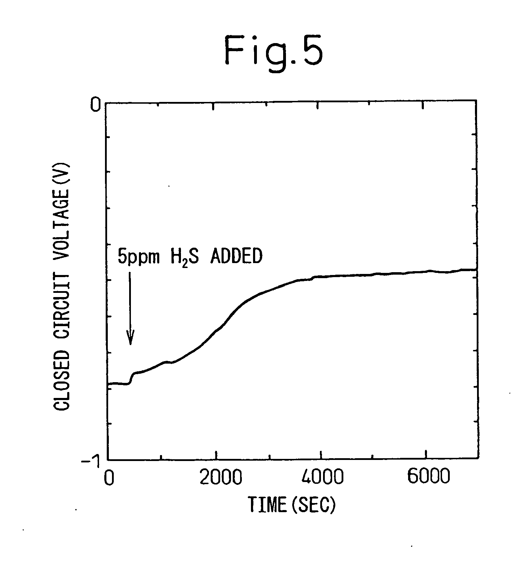

In contrast, in the sulfur-detecting sensors using copper, zinc oxide, tin oxide and silver, the measurements of which are shown in Figs. 5 to 8, respectively, the closed circuit voltages were gradually increased (the potential differences were decreased) with the addition of H₂S to the stream of hydrogen gas, although the degrees of increase were varied depending on the respective sensors. It is inferred that these were caused by the occurrence of a sulfide or the like in the detecting electrode taking a long time to reach equilibrium and the occurred sulfide or the like being hard to lose.

Thus, the sulfur-detecting sensors showing the characteristics represented in Figs. 5 to 8 are suitable for a sensor for measuring the accumulated amount of H₂S in a stream of hydrogen gas having passed over the sensor.

### Example 3

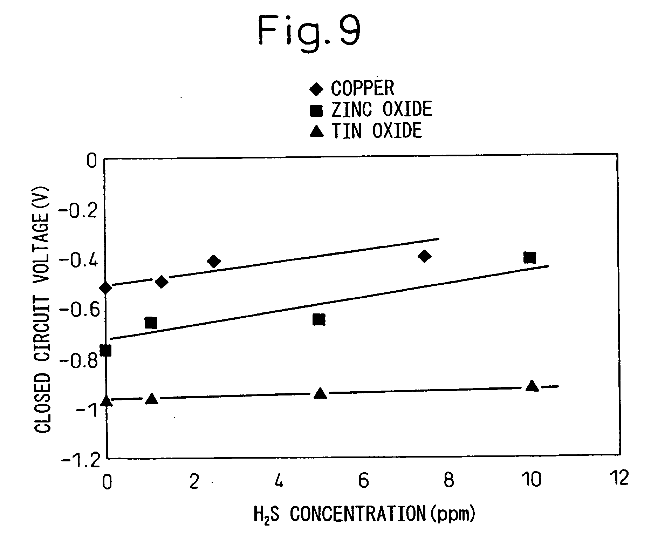

A potential difference between the detecting electrode and the reference electrode of each of the sulfur-detecting sensors using copper, zinc oxide and tin oxide, the measurements of which are shown in Figs. 5 to 7, respectively, was measured under the same conditions as those in Example 1 except that the concentration of H₂S in the H₂S-containing hydrogen gas stream was changed. The results are shown in Fig. 9. However, the values of potential difference between the detecting electrode and the reference electrode (closed circuit voltage) shown in Fig. 9 were obtained 10 minutes after the initiation of introduction of H₂S in the hydrogen gas stream.

As is evident from Fig. 9, the higher the H₂S concentration in the hydrogen gas stream, the more the potential difference between the detecting electrode and the reference electrode is decreased.

### Example 4

Using sulfur-detecting sensors S similar to that of Example 1, sulfur-detecting devices as shown in Fig. 2 were fabricated. In the respective sulfur-detecting sensors, the detecting electrodes were made from, in addition to platinum, rhodium oxide, zinc oxide, and tin oxide, respectively. In each of the fabricated sulfur-detecting devices, the detecting electrode 12 and the reference electrode 14 of the sulfur-detecting sensor S were connected with a voltmeter 22 through wires 16 and 18 of platinum, respectively, and a constant-current generating unit 31 was not provided.

The sulfur-detecting sensor S was installed into a pipe 30 as shown in Fig. 2, and was placed in an electric furnace (not shown) along with the pipe 30. A stream of hydrogen gas (hydrogen gas concentration of 4% by volume, and nitrogen gas concentration of 96% by volume) was introduced in the pipe 30 in the direction of the arrow A to be passed over the detecting electrode 12, and air was passed over the reference electrode 14 outside the pipe 30, while the atmosphere in the furnace was heated to 600°C at which the substrate 10 develops ion conductivity.

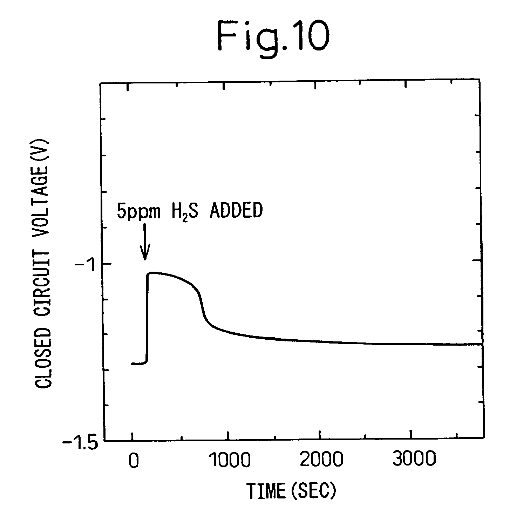

Figs. 10 to 12 show open circuit voltages measured as the potential differences between the detecting electrode 12 and the reference electrode 14 by the voltmeter 22 under the above conditions. The data in Figs. 10 to 12 were obtained from the sensors having the detecting electrodes of platinum and rhodium oxide, platinum and zinc oxide, and platinum and tin oxide, respectively.

As shown in Fig. 10, in the sulfur-detecting sensor having the detecting electrode 12 formed from platinum and rhodium oxide, the open circuit voltage was dramatically decreased (the potential difference was decreased) with the introduction of H2S to the hydrogen gas stream, but was gradually restored after a lapse of a certain time while the introduction of H₂S to the hydrogen gas stream was continued. It is inferred that, in the sulfur-detecting sensor, the measured result of which is shown in Fig. 10, although a sulfide or the like rapidly occurred in the detecting electrode 12 with the introduction of H₂S in the hydrogen gas stream, the resultant sulfide or the like was rapidly lost after a lapse of a certain time, resulting in the gradual restoration of the electrochemical reaction of hydrogen with oxygen ion (O²⁻).

Thus, the sulfur-detecting sensor, the measured result of which is shown in Fig. 10, is suitable for a sensor for measuring the change in concentration of H₂S in a stream of hydrogen gas.

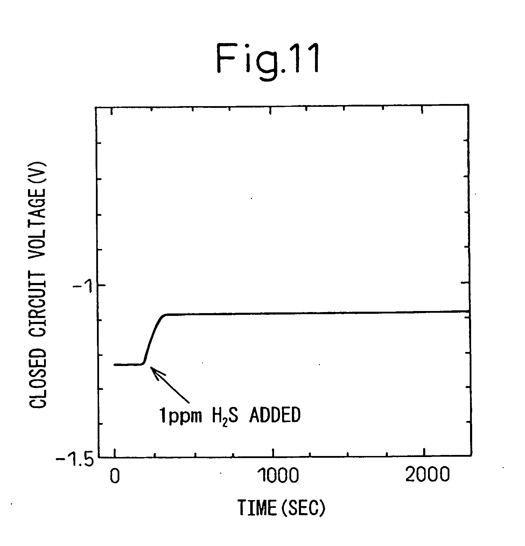

On the other hand, in the sulfur-detecting sensor having the detecting electrode 12 formed from platinum and zinc oxide or tin oxide, the open circuit voltage was gradually increased (the potential difference was decreased) with the introduction of H₂S to the hydrogen gas stream, as shown in Fig. 11 or 12. It is inferred that this was caused by the occurrence of a sulfide or the like in the detecting electrode taking a long time to reach equilibrium and the occurred sulfide or the like being hard to lose.

Thus, the sulfur-detecting sensors, the measured results of which are shown in Figs. 11 and 12, is suitable for applications in which an accumulated amount of H₂S in a hydrogen gas stream having passed over the sensor is measured.

As described, the sulfur-detecting sensor and the sulfur-detecting device of the invention can make it possible to measure an amount of sulfurous component in a substantially oxygen-free gas stream, which could not be measured by a prior sulfur-detecting sensor. Thus, using the sensor and device according to the invention, an amount of sulfurous component in a gas stream can be accumulatively measured in a process for reforming gasoline or the like to produce a hydrogen gas stream to be fed to a fuel cell.

In addition, the sulfur-detecting sensor and the sulfur-detecting device of the invention are compact, and can be employed in a reforming process for a fuel cell for domestic or automobile use.

## Claims

1. A sulfur-detecting sensor for detecting a sulfurous component contained in a substantially oxygen-free gas stream, the sensor comprising a solid electrolyte substrate (10) through which oxygen ions can move, and electrodes (12, 14) for detecting a difference in potential induced by a electrochemical reaction of oxygen ion moving through the solid electrolyte substrate (10) with a component constituting the gas stream, which electrodes (12, 14) for detecting a difference in potential include a detecting electrode (12) formed on one side of the solid electrolyte substrate (10) and brought into contact with the gas stream, and a reference electrode (14) formed on the other side of the solid electrolyte substrate (10) and brought into contact with an oxygen-containing fluid, and **characterized by** the detecting electrode (12) being formed from a metal selected from the group consisting of Ni, Cu, and Zn, or an oxide thereof.

2. The sulfur-detecting sensor of claim 1, wherein the detecting electrode (12) is formed from a transition metal having an enthalpy of formation of oxide of not smaller than -650 kJ/mol at 25°C, or an oxide thereof.

3. The sulfur-detecting sensor of any of the preceding claims wherein the detecting electrode (12) contains platinum.

4. The sulfur-detecting sensor of any of the preceding claims, wherein the reference electrode (14) contains platinum.

5. The sulfur-detecting sensor of any of the preceding claims, wherein the solid electrolyte substrate (10) is a sintered body of yttria-stabilized zirconia, a sintered body of scandia-stabilized zirconia, a sintered body of ceria to which samaria is added, or a sintered body of scandia-stabilized zirconia to which ceria is added.

6. A sulfur-detecting device for detecting a sulfurous component contained in a substantially oxygen-free gas stream, the device comprising a sulfur-detecting sensor, which comprises a solid electrolyte substrate (10) through which oxygen ions can move, and electrodes (12, 14) for detecting a difference in potential induced by a electrochemical reaction of oxygen ions moving through the solid electrolyte substrate (10) with a component constituting the gas stream, the electrodes (12, 14) for detecting a difference in potential including a detecting électrode (12) formed on one side of the solid electrolyte substrate (10) and brought into contact with the gas stream, and a reference electrode (14) formed on the other side of the solid electrolyte substrate (10) and brought into contact with an oxygen-containing fluid, the detecting electrode (12) being formed from a metal selected from the group consisting of Ni, Cu, and Zn, or an oxide thereof at , and the device further comprising a voltmeter for measuring a potential difference between the detecting electrode and the reference electrode of the sulfur-detecting sensor.

7. The sulfur-detecting device of claim 6, which further comprises a constant-current generating unit connected with a closed circuit formed of the solid electrolyte substrate (10), the detecting electrode (12), the reference electrode (14) and the voltmeter, the constant-current generating unit supplying a current to the circuit so that the current density in the detecting electrode (12) is constant.

8. The sulfur-detecting device of claim 6 or 7, wherein the solid electrolyte substrate (10), the detecting electrode (12), the reference electrode (14) and the voltmeter form an open circuit to which a current is not externally supplied.

9. The sulfur-detecting device of one of claims 6 to 8, wherein
the detecting electrode (12) is formed from a transition metal having an enthalpy of formation of oxide of not smaller than -650 kJ/mol at 25°C, or an oxide thereof.

10. The sulfur-detecting device of one of claims 6 to 9, wherein the detecting electrode (12) contains platinum.

11. The sulfur-detecting device of one of claims 6 to 10 , wherein the reference electrode (14) contains platinum.

12. The sulfur-detecting device of one of claims 6 to 11, wherein the solid electrolyte substrate (10) is a sintered body of yttria-stabilized zirconia, a sintered body of scandia-stabilized zirconia, a sintered body of ceria to which samaria is added, or a sintered body of scandia-stabilized zirconia to which ceria is added.

## Patentansprüche

1. Schwefel-Nachweissensor zur Detektion einer schwefelhaltigen Komponente, die in einem im Wesentlichen sauerstofffreien Gasstrom enthalten ist, wobei der Sensor umfasst:
ein Festelektrolytsubstrat (10), durch das Sauerstoffionen wandern können, und Elektroden (12, 14) zum Detektieren einer Potentialdifferenz, die durch eine elektrochemische Reaktion von durch das Festelektrolytsubstrat (10) wandernden Sauerstoffionen mit einer den Gasstrom bildenden Komponente induziert ist, wobei die Elektroden (12, 14) zum Detektieren einer Potentialdifferenz eine Detektorelektrode (12), die auf einer Seite des Festelektrolytsubstrats (10) ausgebildet ist und mit dem Gasstrom in Kontakt gebracht wird, und eine Referenzelektrode (14) aufweisen, die auf der anderen Seite des Festelektrolytsubstrats (10) ausgebildet ist und mit einem sauerstoffhaltigen Fluid in Kontakt gebracht wird,
und **gekennzeichnet dadurch, dass** die Detektorelektrode (12) aus einem Metall gebildet ist, das ausgewählt ist aus der Gruppe bestehend aus Ni, Cu und Zn oder einem Oxid davon.

2. Schwefel-Nachweissensor nach Anspruch 1, bei dem die Detektorelektrode (12) aus einem Übergangsmetall, das eine Oxidbildungsenthalpie von nicht weniger als -650 kJ/mol bei 25 °C aufweist, oder einem Oxid davon gebildet ist.

3. Schwefel-Nachweissensor nach einem der vorhergehenden Ansprüche, bei dem die Detektorelektrode (12) Platin enthält.

4. Schwefel-Nachweissensor nach einem der vorhergehenden Ansprüche, bei dem die Referenzelektrode (14) Platin enthält.

5. Schwefel-Nachweissensor nach einem der vorhergehenden Ansprüche, bei dem das Festelektrolytsubstrat (10) als Sinterkörper aus mit Yttriumoxid stabilisiertem Zirconiumoxid, als Sinterkörper aus mit Scandiumoxid stabilisiertem Zirconiumoxid, als Sinterkörper aus Ceroxid mit zugesetztem Samariumoxid oder als Sinterkörper aus mit Scandiumoxid stabilisiertem Zirconiumoxid mit zugesetztem Ceroxid gebildet ist.

6. Schwefel-Nachweiseinrichtung zur Detektion einer schwefelhaltigen Komponente, die in einem im Wesentlichen sauerstofffreien Gasstrom enthalten ist, wobei die Einrichtung einen Schwefel-Nachweissensor umfasst, der umfasst:
ein Festelektrolytsubstrat (10), durch das Sauerstoffionen wandern können, und Elektroden (12, 14) zum Detektieren einer Potentialdifferenz, die durch eine elektrochemische Reaktion von durch das Festelektrolytsubstrat (10) wandernden Sauerstoffionen mit einer den Gasstrom bildenden Komponente induziert ist, wobei die Elektroden (12, 14) zum Detektieren einer Potentialdifferenz eine Detektorelektrode (12), die auf einer Seite des Festelektrolytsubstrats (10) ausgebildet ist und mit dem Gasstrom in Kontakt gebracht wird, und eine Referenzelektrode (14) aufweisen, die auf der anderen Seite des Festelektrolytsubstrats (10) ausgebildet ist und mit einem sauerstoffhaltigen Fluid in Kontakt gebracht wird, wobei die Detektorelektrode (12) aus einem Metall gebildet ist, das ausgewählt ist aus der Gruppe bestehend aus Ni, Cu und Zn oder einem Oxid davon, und die Einrichtung weiter ein Voltmeter zum Messen einer Potentialdifferenz zwischen der Detektorelektrode und der Referenzelektrode des Schwefel-Nachweissensors umfasst.

7. Schwefel-Nachweiseinrichtung nach Anspruch 6, die weiter eine Konstantstromgeneratoreinheit umfasst, die mit einem geschlossenen Schaltkreis verbunden ist, der durch das Festelektrolytsubstrat (10), die Detektorelektrode (12), die Referenzelektrode (14) und das Voltmeter gebildet ist, wobei die Konstantstromgeneratoreinheit dem Schaltkreis einen Strom so zuführt, dass die Stromdichte in der Detektorelektrode (12) konstant ist.

8. Schwefel-Nachweiseinrichtung nach Anspruch 6 oder 7, bei der das Festelektrolytsubstrat (10), die Detektorelektrode (12), die Referenzelektrode (14) und das Voltmeter einen offenen Schaltkreis bilden, dem kein Strom von außen zugeführt wird.

9. Schwefel-Nachweiseinrichtung nach einem der Ansprüche 6 bis 8, bei der die Detektorelektrode (12) aus einem Übergangsmetall, das eine Oxidbildungsenthalpie von nicht weniger als -650 kJ/mol bei 25 °C aufweist, oder einem Oxid davon gebildet ist.

10. Schwefel-Nachweiseinrichtung nach einem der Ansprüche 6 bis 9, bei der die Detektorelektrode (12) Platin enthält.

11. Schwefel-Nachweiseinrichtung nach einem der Ansprüche 6 bis 10, bei der die Referenzelektrode (14) Platin enthält.

12. Schwefel-Nachweiseinrichtung nach einem der Ansprüche 6 bis 11, bei der das Festelektrolytsubstrat (10) als Sinterkörper aus mit Yttriumoxid stabilisiertem Zirconiumoxid, als Sinterkörper aus mit Scandiumoxid stabilisiertem Zirconiumoxid, als Sinterkörper aus Ceroxid mit zugesetztem Samariumoxid oder als Sinterkörper aus mit Scandiumoxid stabilisiertem Zirconiumoxid mit zugesetztem Ceroxid gebildet ist.

## Revendications

1. Capteur de détection du soufre destiné à détecter un composant soufré présent dans un écoulement gazeux essentiellement dépourvu d'oxygène, le capteur comprenant
un substrat (10) en électrolyte solide qui peut être traversé par les ions oxygène et des électrodes (12, 14) qui détectent une différence de potentiel induite par la réaction électrochimique de l'ion oxygène traversant le substrat (10) d'électrolyte solide avec un composant constituant l'écoulement de gaz, les électrodes (12, 14) servant à détecter une différence de potentiel comprenant une électrode de détection (12) formée sur un côté du substrat (10) d'électrolyte solide et mise en contact avec l'écoulement de gaz et une électrode de référence (14) formée sur l'autre côté du substrat (10) d'électrolyte solide et mise en contact avec un fluide contenant de l'oxygène, et **caractérisé par** l'électrode de détection (12) étant formée d'un métal sélectionné dans l'ensemble constitué de Ni, Cu et Zn ou d'un de leurs oxydes.

2. Capteur de détection du soufre selon la revendication 1, l'électrode de détection (12) étant formée d'un métal de transition dont l'enthalpie de formation d'oxyde n'est pas inférieure à -650 kJ/mole à 25°C, ou d'un oxyde de ce métal.

3. Capteur de détection du soufre selon l'une quelconque des revendications précédentes, dans lequel l'électrode de détection (12) contient du platine.

4. Capteur de détection du soufre selon l'une quelconque des revendications précédentes, dans lequel l'électrode de référence (14) contient du platine.

5. Capteur de détection du soufre selon l'une quelconque des revendications précédentes, dans lequel le substrat (10) d'électrolyte solide est un corps fritté en zirconia stabilisé par yttria, un corps fritté de zirconia stabilité par scandia, un corps fritté de céria auquel est ajoutée de la samaria ou un corps fritté de zirconia stabilisé par scandia et auquel de la céria est ajoutée.

6. Dispositif de détection du soufre servant à détecter un composant soufré présent dans un écoulement gazeux essentiellement dépourvu d'oxygène, le dispositif comprenant un capteur de détection du soufre qui comprend un capteur détectant le soufre qui comprend
un substrat (10) en électrolyte solide qui peut être traversé par les ions oxygène et des électrodes (12, 14) qui détectent une différence de potentiel induite par la réaction électrochimique de l'ion oxygène traversant le substrat (10) d'électrolyte solide avec un composant constituant l'écoulement de gaz, les électrodes (12, 14) servant à détecter une différence de potentiel comprenant une électrode de détection (12) formée sur un côté du substrat (10) d'électrolyte solide et mise en contact avec l'écoulement de gaz et une électrode de référence (14) formée sur l'autre côté du substrat (10) d'électrolyte solide et mise en contact avec un fluide contenant de l'oxygène, l'électrode de détection (12) étant formée d'un métal sélectionné dans l'ensemble constitué de Ni, Cu et Zn ou d'un de leurs oxydes, le dispositif comprenant en outre un voltmètre qui mesure une différence de potentiel entre l'électrode de détection et l'électrode de référence du capteur de détection du soufre.

7. Dispositif de détection du soufre selon la revendication 6, comprenant en outre une unité de production de courant constant raccordée à un circuit fermé formée par le substrat (10) d'électrolyte solide, l'électrode de détection (12), l'électrode de référence (14) et le voltmètre, l'unité de production d'un courant constant délivrant un courant au circuit de telle sorte que la densité de courant dans l'électrode de détection (12) soit constante.

8. Dispositif de détection du soufre selon les revendications 6 ou 7, dans lequel le substrat (10) d'électrolyte solide, l'électrode de détection (12), l'électrode de référence (14) et le voltmètre forment un circuit ouvert auquel aucun courant n'est délivré depuis l'extérieur.

9. Dispositif de détection du soufre selon l'une des revendications 6 à 8, dans lequel l'électrode de détection (12) est formée d'un métal de transition dont l'enthalpie de formation d'oxyde n'est pas inférieure à -650 kJ/mole à 25°C, ou d'un oxyde de ce métal.

10. Dispositif de détection du soufre selon l'une des revendications 6 à 9, dans lequel l'électrode de détection (12) contient du platine.

11. Dispositif de détection du soufre selon l'une des revendications 6 à 10, dans lequel l'électrode de référence (14) contient du platine.

12. Dispositif de détection du soufre selon l'une des revendications 6 à 11, dans lequel le substrat (10) d'électrolyte solide est un corps fritté en zirconia stabilisé par yttria, un corps fritté de zirconia stabilité par scandia, un corps fritté de céria auquel est ajoutée de la samaria ou un corps fritté de zirconia stabilisé par scandia et auquel de la céria est ajoutée.
